(19) Europäisches Patentamt
European Patent Office
Office européen des brevets

(11) Publication number: **0 430 129 A2**

(12) # EUROPEAN PATENT APPLICATION

(21) Application number: **90122525.0**

(22) Date of filing: **26.11.90**

(51) Int. Cl.5: C07C 59/56, C07C 59/64, C07C 69/732, C07D 333/00, C07D 307/00

A REQUEST FOR CORRECTION HAS BEEN FILED PURSUANT TO RULE 88 EPC. A DECISION ON THE REQUEST WILL BE TAKEN DURING THE PROCEEDINGS BEFORE THE EXAMINING DIVISION (GUIDELINES FOR EXAMINATION IN THE EPO, A-V, 2.2).

(30) Priority: **30.11.89 JP 313053/89**
**06.04.90 JP 92551/90**

(43) Date of publication of application:
**05.06.91 Bulletin 91/23**

(84) Designated Contracting States:
**AT BE CH DE DK ES FR GB GR IT LI LU NL SE Bulletin**

(71) Applicant: **TANABE SEIYAKU CO., LTD.**
**2-10, Dosho-machi 3-chome**
**Chuo-ku Osaka(JP)**

(72) Inventor: **Nakai, Hideo**
**No. 19-18, Hanayashiki-matsugaoka**
**Takarazuka-shi, Hyogo-ken(JP)**
Inventor: **Tanaka, Takashi**
**No. 1685-1, Oaza-Nakao**
**Urawa-shi, Saitama-ken(JP)**
Inventor: **Nomura, Sumihiro**
**No. 4-11-509, Hikonari 4-chome**
**Misato-shi, Saitama-ken(JP)**
Inventor: **Takashima, Kohki**
**No. 1-3-904, Higashi-kasai 5-chome,**
**Edogawa-ku**
**Tokyo-to(JP)**
Inventor: **Suzuki, Kazuko**
**304 Fujiwara Mansion, No. 46-15,**
**Kitazono-cho**
**Kawaguchi-shi, Saitama-ken(JP)**

(74) Representative: **Hansen, Bernd, Dr. Dipl.-Chem. et al**
**Hoffmann, Eitle & Partner Patent- und Rechtsanwälte Arabellastrasse 4**
**W-8000 München 81(DE)**

(54) **Novel phenylethylene derivatives, processes for preparing the same and intermediates therefor.**

(57) A novel phenylethylene derivative of the formula:

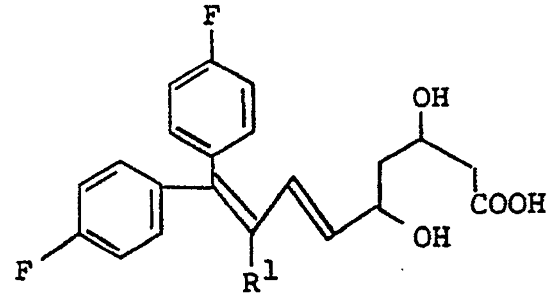

wherein $R^1$ is phenyl group, a fluorophenyl group, a methoxyphenyl group, thienyl group, furyl group, oxopyrrolidinyl group or pyridyl group, or a pharmaceutically acceptable ester, amide, lactone or salt, which has excellent HMG-CoA reductase inhibitory activity and is useful as an anti-hyperlipidemic agent, a pharmaceutical composition containing the same, and processes for preparing the same as well as an intermediate therefor.

EP 0 430 129 A2

# NOVEL PHENYLETHYLENE DERIVATIVES, PROCESSES FOR PREPARING THE SAME AND INTERMEDIATES THEREFOR

The present invention relates to novel phenylethylene derivatives which are useful as an anti-hyperlipidemic agent. The compounds of the present invention exhibit anti-hyperlipidemic activity thereof through inhibition of HMG-CoA reductase.

## Prior Art

Hyperlipidemia is considered to be one of main factors causing arteriosclerosis which is one of adult diseases. There have been known various agents for treatment and prophylaxis of hyperlipidemia such as clofibrate [chemical nomenclature; 2-(4-chlorophenoxy)-2-methylpropanoic acid ethyl ester], probucol [chemical nomenclature; 4,4'-[(1-methylethylidene)bis(thio)]bis[2,6-bis(1,1-dimethylethyl)phenol]] and the like, which exhibit the anti-hyperlipidemic activity thereof through inhibition of absorption of cholesterol and bile acid, or inhibition of synthesis and secretion of very low density lipoprotein (VLDL).

On the other hand, it is known that 3-hydroxy-3-methylglutaryl coenzyme A reductase (hereinafter referred to as HMG-CoA reductase) catalyzes the biosynthesis system wherein 3-hydroxy-3-methylglutaryl coenzyme A (hereinafter referred to as HMG-CoA) is converted into a precursor of cholesterol; mevalonic acid. It is, therefore, considered that cholesterol biosynthesis can be inhibited by inhibition of HMG-CoA reductase, and there is desired to develop a novel anti-hyperlipidemic agent which can exhibit an anti-hyperlipidemic activity based on the above-mentioned mechanism.

As a compound which has HMG-CoA reductase inhibitory activity, there have been known 3,5-dihydroxycarboxylic acid derivatives such as sodium 9,9-bis(4-fluorophenyl)-3,5-dihydroxy-8-isopropyl-6,8-nonadienoate [Japanese Patent Publication (unexamined) No. 45337/1989].

## Brief Description of the Invention

An object of the present inventicn is to provide a novel phenylethylene derivative which has excellent HMG-CoA reductase inhibitory activity and is useful as an anti-hyperlipidemic agent.

Another object of the invention is to provide a process for preparing said novel compound.

A further object of the invention is to provide an intermediate useful for preparing said novel compound.

## Detailed Description of the Invention

The present invention relates to a novel phenylethylene derivative of the formula:

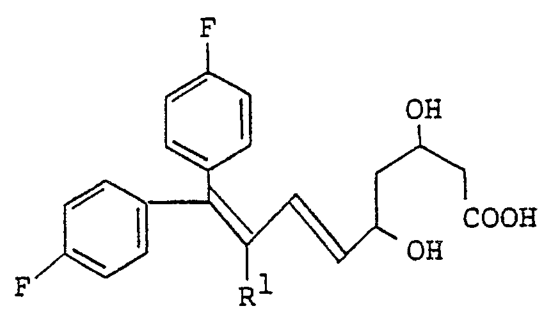

(I)

wherein $R^1$ is phenyl group, a fluorophenyl groups, a methoxyphenyl group, thienyl group, furyl group, oxopyrrlidinyl group or pyridyl group, or a pharmaceutically acceptable ester, amide, lactone or salt thereof.

The desired compound (I) of the present invention has an excellent HMG-CoA reductase inhibitory activity, and hence, is useful as an anti-hyperlipidemic agent, especially as agents for prophylaxis and treatment of hypercholesterolemia.

The desired compound (I) of the present invention can be used in either form of a free carboxylic acid, or a form of a pharmaceutically acceptable ester, amide, lactone, or salt thereof in therapeutical field.

An ester of the compound (I) includes a lower alkyl ester, a phenyl-lower alkyl ester. An amide of the compound (I) includes a non-substituted amide, a mono-lower alkyl amide, a di-lower alkyl amide. A lactone of the compound (I) includes the compound of the formula:

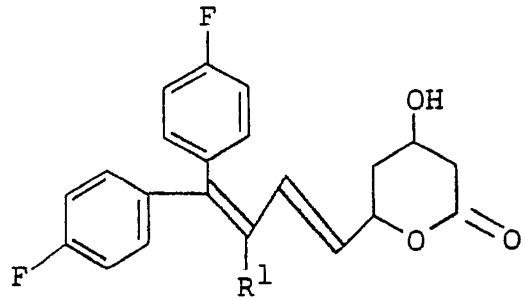

(I-a)

wherein $R^1$ is the same as defined above.

Through the present specification and claims, the term "lower alkyl" denotes an alkyl having 1 to 4 carbon atoms.

A pharmaceutically acceptable salt of the compound (I) includes an alkali metal salt (e.g. sodium salt, potassium salt, etc.), an alkaline earth metal (e.g. calcium salt, magnesium salt, etc.), a heavy metal salt (e.g. zinc salt, etc.), and a salt with an organic amine (e.g. ammonium salt, triethylamine salt, pyridine salt, ethanolamine salt, basic amino acid salt, etc.).

The compound (I) of the present invention includes four optical isomers based on two asymmetric carbon atoms and a mixture thereof, and when the compound (I) of the present invention is other than a lactone compound, (3R,5S)-isomer thereof is most preferable as a medicament, and when the compound (I) of the present invention is a lactone compound (I-a), (4R,6S)-isomer is most preferable as a medicament.

The compound (I) of the present invention can be prepared, for example, by reacting an aldehyde compound of the formula:

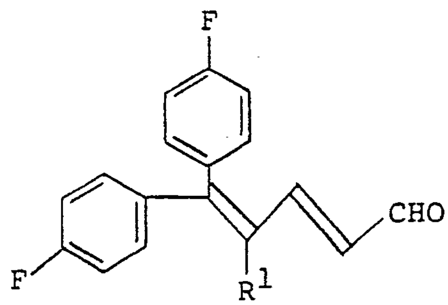

(II)

wherein $R^1$ is the same as defined above, or a salt thereof with an acetoacetic acid compound of the formula:

$CH_3COCH_2COOR^2$ (III)

wherein a group of the formula: $-COOR^2$ is a carboxyl group which may optionally have a protecting group, or a salt thereof, reducing the resulting intermediate (IV) of the formula:

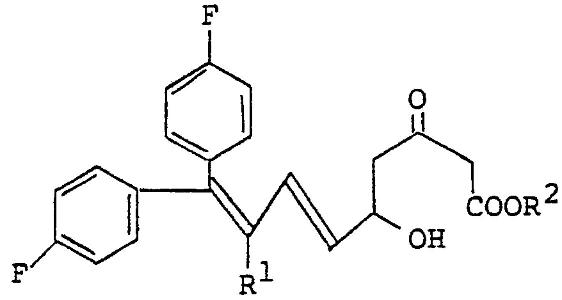

(IV)

wherein $R^1$ and a group of the formula: $-COOR^2$ are the same as defined above, or a salt thereof, and removing optionally the protecting group thereof when a group of the formula: $-COOR^2$ is a carboxyl group having a protecting group.

The protecting group for the acetoacetic acid compound (III) and the intermediate (IV) may be any protecting group which can easily be removed by a conventional method such as hydrolysis, reduction, solvolysis, acid treatment and the like, and includes, for example, a lower alkyl group, a substituted or unsubstituted phenyl-lower alkyl group (e.g. benzyl group, p-methoxybenzyl group, p-nitrobenzyl group, etc.), benzhydryl group, and the like.

The reaction between the aldehyde compound (II) or a salt thereof with the acetoacetic acid compound (III) or a salt thereof is preferably carried out in the presence of a base. The base includes, for example, an alkali metal hydride, a lower alkyllithium (e.g. n-butyllithium, etc.), lithium diisopropylamide, and the like. In this reaction, the aldehyde compound (II) can also be used in the form of a salt thereof with a conventional organic acid or inorganic acid, and the acetoacetic acid compound (III), wherein a group of the formula: $-COOR^2$ is a free carboxyl group, can be used in the form of a salt thereof with a conventional organic base or inorganic base. The present reaction is preferably carried out at a temperature of -78 °C to room temperature.

The reduction of the intermediate compound (IV) can be carried out using a reducing agent which can reduce selectively the ketone moiety of the compound (IV), for example, by using an alkali metal borohydride, or a combination of a boron compound such as tri-lower alkylboron (e.g. triethylboron, tri(n-butyl)boron, etc.), or di-lower alkyl-lower alkoxyboron (e.g. diethylmethoxyboron, etc.) with an alkali metal borohydride. When the intermediate compound (IV) is a free carboxylic acid, it may be used in the form of an alkali metal salt thereof. This reaction is preferably carried out in a suitable solvent (e.g. ether, tetrahydrofuran, dioxane, and a mixture thereof) at a temperature of -78 °C to room temperature.

When the group of the formula: $-COOR^2$ is a carboxyl group having a protecting group, the removing of the protecting group: $R^2$ from the product obtained in the above reduction can be carried out by a conventional method such as hydrolysis, reduction, solvolysis, acid treatment, and the like, for example, the compound (I) in the form of a free carboxylic acid can be obtained by hydrolysis of the reduction product in the presence of a base, followed by neutralization of the product thereof with an acid. The base used in above is, for example, an alkali metal hydroxide, an alkali metal carbonate, an alkali metal hydrogen carbonate, and the like, and the acid used in above is, for example, an inorganic acid such as hydrochloric acid and the like. This reaction is preferably carried out at room temperature to 100 °C.

The ester, amide or lactone of the compound (I) can be prepared from the compound (I) in the form of a free carboxylic acid by a conventional method. For example, the ester of the compound (I) can be prepared by reacting the compound (I) in the form of a free carboxylic acid with a lower alkanol, or a phenyl-lower alkanol in the presence of an acid catalyst (e.g. hydrogen chloride, sulfuric acid, p-toluenesulfonic acid, strong acid ion-exchange resin, etc.). The amide of the compound (I) can be prepared by reacting the compound (I) in the form of a free carboxylic acid with ammonia, a lower alkylamine or a di-lower alkylamine. The lactone compound (I-a) can be prepared by heating the compound (I) in the form of a free carboxylic acid. These reactions are preferably carried out in a solvent such as alkanol, toluene, and the like.

The compound (I) of the present invention can, if desired, be resolved into corresponding optical isomers thereof by optical resolution. The resolving agent may be any conventional one, such as optically active 1-phenylethylamine, 1-(2-naphthyl)ethylamine, α-methyl-p-nitrobenzylamine, 1-(1-naphthyl)-ethylamine, and the like. For example, by reacting (±)-trans-(E)-6-[4,4-bis(4-fluorophenyl)-3-(2-thienyl)-1,3-butadienyl]-3,4,5,6-tetrahydro-4-hydroxy-2H-pyran-2-one with (R)-(+)-1-phenylethylamine, separating the resulting two diasteromers by column chromatography, subjecting to hydrolysis, and then acidifying, there is obtained the corresponding optically active compound (I) in the form of a free carboxylic acid. If desired, this compound is heat-treated to give an optically active lactone compound.

The compound (I) of the present invention, and a pharmaceutically acceptable ester, amide, lactone and salt thereof have an excellent activity for inhibiting HMG-CoA reductase, and hence are useful as anti-hyperlipidemic agents and can be used for treatment, amelioration and prophylaxis of hyperlipidemia, coronary disease, arteriosclerosis, familial hypercholesterolemia and xanthoma, and the other related diseases.

The compound (I) of the present invention, and a pharmaceutically acceptable ester, amide, lactone and salt can be administered either orally or parenterally to a warmblooded animal, including human beings, by formulating into conventional pharmaceutical preparations tablets, granules, capsules, powders, injections and the like.

The dosage of the compound (I) of the present invention may vary depending on an administration route, age, weight and conditions of the patients, but it is usually in the range of about 0.05 - 10 mg/kg per day, preferably 0.1 - 5 mg/kg per day.

The starting compound (II) of the present invention is a novel compound, and can be prepared by

preparing the compound of the formula:

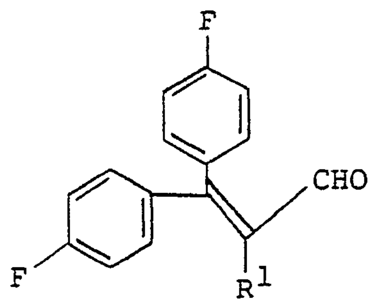

(V)

wherein $R^1$ is the same as defined above according to the method disclosed in J.A.C.S. 73, 2716 (1951), and reacting the compound (V) with N-ethylidenecyclohexaneamine and lithium diisopropylamide, or reacting the compound (V) with 2-[bis(1-methylethoxy)phosphinyl]acetonitrile in the presence of sodium hydride, followed by reducing the product, or reacting the compound (V) with diethyl 2-(cyclohexylamino)-vinylphosphonate in the presence of sodium hydride, followed by acidifying the product.

Experiment: Inhibitory activity against Rat Hepatic Microsomal HMG-CoA reductase (HMGR)

HMGR activities of microsomes are measured in accordance with the method of N. L. Young et al., Methods in Enzymology, 71, 498 (1981).

Microsomes prepared from liver of rats administered with cholestyramine were mixed with a solution of the compounds to be tested in dimethyl sulfoxide and $^{14}C$-HMG-CoA (substrate). After the mixture was incubated for 10 minutes, the reaction was quenched with 6N hydrochloric acid and the mixture was allowed to stand at 37° C for 15 minutes, followed by centrifugation. The supernatant was spotted on thin layer chromatography (TLC; Kieselgel 60F254 manufactured by Merck) and developed with toluene/acetone (1:1). The spot of $^{14}C$-mevalonolactone was collected and the amount of isotope was measured with a scintillation counter (Type 4640 TRI-CARB. manufactured by Paccard) to calculate an amount of the formed mevalonolactone. By comparing amounts of the formed mevalonolactone in the group treated with test compounds and the control group (without test compounds), HMGR activity inhibition rates of the test compounds were calculated. The results are shown in Table 1.

Table 1 HMGR inhibition (%)

| 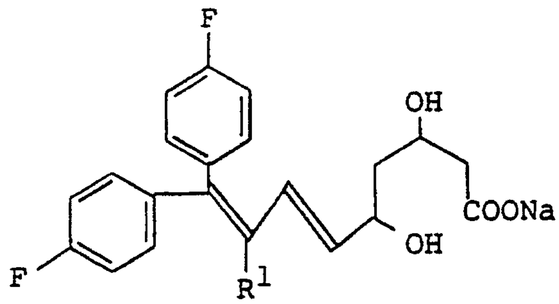 | | Concentrations of of Test compounds (M) | |
|---|---|---|---|
| $R^1$ | Isomer | $10^{-4}$ | $10^{-6}$ |
| 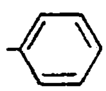 | (±)-(3R*,5S*) | 96 | 79 |
| 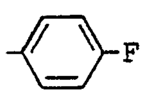 | (±)-(3R*,5S*) | 96 | - |
| 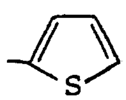 | (+)-(3R*,5S*) | - | 88 |
| 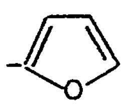 | (-)-(3R*,5S*) | - | 93 |

Example 1

(1) To a solution of (E)-5,5-bis(4-fluorophenyl)-4-phenyl-2,4-pentadienenitrile (900 mg) in tetrahydrofuran (10 ml) is added a 1.5 M solution of diisobutylaluminum hydride in toluene (7.0 ml), and the mixture is stirred at 0°C for 15 minutes. Thereto is added 10 % hydrochloric acid, and the mixture is extracted with ethyl acetate. The solvent is distilled off, and the resulting residue is purified and separated by silica gel column chromatography (solvent; n-hexane : ethyl acetate = 20 : 1) to give (E)-5,5-bis(4-fluorophenyl)-4-phenyl-2,4-pentadienal (600 mg) as yellow crystal.
Yield: 66 %
M.p.: 120 - 123°C
MS (m/z): 346 ($M^+$)

$$IR\ \nu^{Nujol}_{Max}\ (cm^{-1}):\ 1670,\ 1590,\ 1460$$

(2) A mixture of 60 % sodium hydride (130 mg), tetrahydrofuran (3 ml) and methyl acetoacetate (380 mg) is stirred at room temperature for 10 minutes under argon atmosphere. After completion of the reaction, the mixture is cooled to -5°C - 0°C, and thereto is added dropwise a 1.6 N solution (2.0 ml) of n-butyllitium in hexane. The mixture is stirred at -5°C for 15 minutes, and thereto is added dropwise a solution of (E)-5,5-bis(4-fluorophenyl)-4-phenyl-2,4-pentadienal (560 mg) in tetrahdyrofuran (7 ml), and the mixture is stirred at -5°C for 15 minutes. After completion of the reaction, ice and a saturated aqueous ammonium chloride solution are added to the mixture. The mixture is extracted with ethyl acetate, and the solvent is distilled off. The resulting residue is purified and separated by silica gel

column chromatography (solvent; n-hexane : ethyl acetate = 2 : 1) to give methyl (E)-9,9-bis(4-fluorophenyl)-5-hydroxy-3-oxo-8-phenyl-6,8-nonadienoate (550 mg) as yellow oil.
Yield: 74 %
MS (m/z): 462 ($M^+$), 346

$$IR\ \nu_{Max}^{Liquid}\ (cm^{-1}):\ 3500,\ 1740,\ 1710,\ 1590,\ 1500$$

(3) A mixture of the product obtained in above (2) (520 mg), tetrahydrofuran (4 ml) and 1 M solution of triethylborane-tetrahydropyran (1.8 ml) is stirred at room temperature under argon atmosphere, and thereto is blown air (1 ml), and the mixture is stirred at room temperature for 10 minutes. The mixture is cooled to -70°C and thereto are added sodium borohydride (85 mg) and methanol (1.1 ml), and the mixture is stirred at the same temperature for 30 minutes. To the mixture is added 30 % aqueous hydrogen peroxide solution in portions, and the mixture is stirred at room temperature for 20 minutes. Water is added thereto, and the mixture is extracted with ethyl acetate. The extract is washed and dried, and the solvent is distilled off. The resulting residue is purified and separated by silica gel column chromatography (solvent; n-hexane : ethyl acetate = 1 : 1) to give methyl (3R*,5S*)-(E)-9,9-bis(4-fluorophenyl)-3,5-dihydroxy-8-phenyl-6,8-nonadienoate (430 mg) as colorless oil.
Yield: 82 %
MS (m/z): 464 ($M^+$), 203

$$IR\ \nu_{Max}^{Liquid}\ (cm^{-1}):\ 3440,\ 1730,\ 1590,\ 1500$$

(4) A mixture of the product obtained in above (3) (410 mg), methanol (1 ml) and 1 N aqueous sodium hydroxide solution (1.1 ml) is stirred at room temperature for 20 minutes. After completion of the reaction, the solvent is distilled off and the resulting residue is purified by a column filled with nonionic absorption resin (trade name; Daiaion HP-20 manufactured by Mitsubishi Kasei Corporation, solvent; methanol : water = 1 : 1) to give sodium (3R*,5S*)-(E)-9,9-bis(4-fluorophenyl)-3,5-dihydroxy-8-phenyl-6,8-nonadienoate (300 mg) as light yellow powder.
Yield: 72 %
FABMS (m/z): 495 ($M^+$+Na), 473 ($M^+$+1), 115

$$IR\ \nu_{Max}^{Nujol}\ (cm^{-1}):\ 3360,\ 1590,\ 1570,\ 1500$$

Examples 2 - 6

(1) The compounds of the following Table 2 are obtained by treating the products of Reference Examples 6 - 10 disclosed hereinafter in the same manner as in Example 1-(1).

Table 2

| Ex. No. | 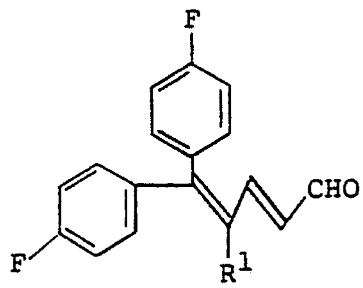 | |
|---|---|---|
| | $R^1$ | Physicochemical Properties |
| 2-(1) | 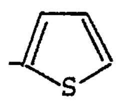 | M.p.: 101-104°C<br>MS (m/z): 352 ($M^+$)<br>IR $\nu_{Max}^{Nujol}$ (cm-1): 1675, 1592, 1122 |
| 3-(1) | 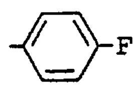 | M.p.: 152-154°C<br>MS (m/z): 364 ($M^+$)<br>IR $\nu_{Max}^{Nujol}$ (cm-1): 1670, 1590, 1500, 1220 |
| 4-(1) | 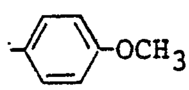 | M.p.: 131-135°C<br>MS (m/z): 376 ($M^+$)<br>IR $\nu_{Max}^{Nujol}$ ($cm^{-1}$): 1671, 1596, 1508, 1463, 1292 |
| 5-(1) | 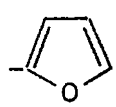 | M.p.: 112-114.5°C<br>MS (m/z): 336 ($M^+$)<br>IR $\nu_{Max}^{Nujol}$ ($cm^{-1}$): 1676, 1597, 1504 |
| 6-(1) | 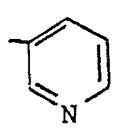 | Caramel<br>MS (m/z): 347 ($M^+$)<br>IR $\nu_{Max}^{Nujol}$ ($cm^{-1}$): 1672, 1597, 1504 |

(2) The compounds of the following Table 3 are obtained by treating the products obtained in above (1) in the same manner as in Example 1-(2).

Table 3

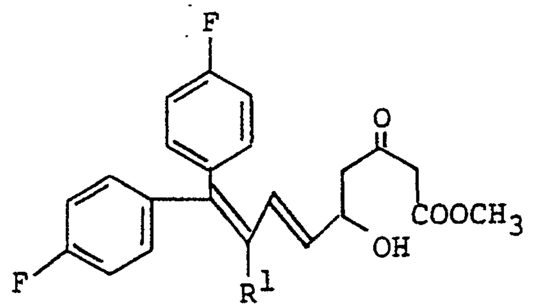

| Ex. No. | $R^1$ | Physicochemical Properties |
|---|---|---|
| 2-(2) | 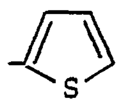 | Oil<br>MS (m/z): 468 ($M^+$), 352<br>IR $\nu_{Max}^{Nujol}$ (cm-1): 3502, 1747, 1716, 1600 |
| 3-(2) | 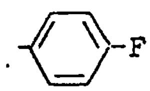 | Oil<br>MS (m/z): 480 ($M^+$), 364<br>IR $\nu_{Max}^{Nujol}$ ($cm^{-1}$): 3480, 1740, 1710 |
| 4-(2) | 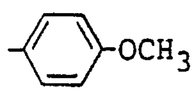 | Oil<br>MS (m/z): 492 ($M^+$), 203<br>IR $\nu_{Max}^{Nujol}$ ($cm^{-1}$): 3900, 1750, 1710, 1600 |
| 5-(2) | 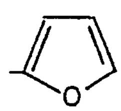 | Oil<br>MS (m/z): 452 ($M^+$), 307<br>IR $\nu_{Max}^{Nujol}$ ($cm^{-1}$): 3504, 1747, 1716, 1601 |
| 6-(2) | 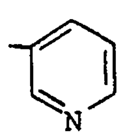 | Oil<br>MS (m/z): 463 ($M^+$), 344<br>IR $\nu_{Max}^{Nujol}$ ($cm^{-1}$): 3600, 1743, 1716 |

(3) The (3R*,5S*)-type compounds of the following Table 4 are obtained by treating the products obtained in above (2) in the same manner as in Example 1-(3).

Table 4

| Ex. No. | 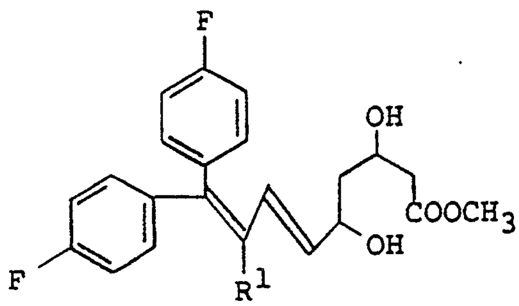 | |
|---|---|---|
| | $R^1$ | Physicochemical Properties |
| 2-(3) | 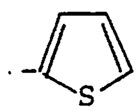 | Oil<br>MS (m/z): 452 ($M^+$-18)<br>IR $\nu^{Nujol}_{Max}$ (cm-1): 3446, 1734, 1600, 1506 |
| 3-(3) | 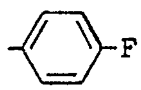 | Oil<br>MS (m/z): 482 ($M^+$)<br>IR $\nu^{Nujol}_{Max}$ ($cm^{-1}$): 3400, 1730, 1600, 1510 |
| 4-(3) | 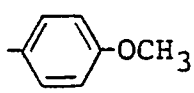 | Oil<br>MS (m/z): 494 ($M^+$)<br>IR $\nu^{Nujol}_{Max}$ ($cm^{-1}$): 3460, 1718, 1602, 1506 |

| | | |
|---|---|---|
| 5-(3) | 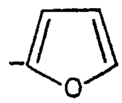 | Oil<br>MS (m/z): 454 ($M^+$)<br>IR $\nu_{Max}^{Nujol}$ ($cm^{-1}$): 3415, 1734, 1601, 1506 |
| 6-(3) | 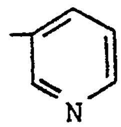 | Oil<br>MS (m/z): 465 ($M^+$)<br>IR $\nu_{Max}^{Nujol}$ ($cm^{-1}$): 3480, 1724, 1600, 1506 |

(4) The (3R*,5S*)-type compounds of the following Table 5 are obtained by treating the products obtained in above (3) in the same manner as in Example 1-(4).

Table 5

| Ex. No. | 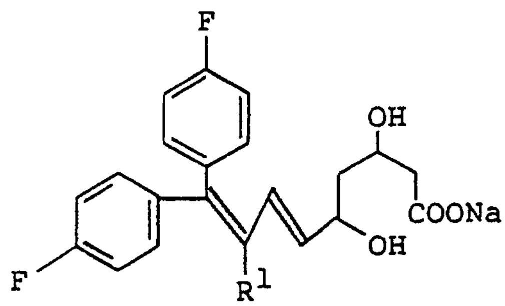 | |
|---|---|---|
| | $R^1$ | Physicochemical Properties |
| 2-(4) | 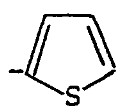 | Powder<br>FABMS (m/z): 501 ($M^+$+Na)<br>IR $\nu_{Max}^{Nujol}$ (cm-1): 3357, 1600, 1572 |
| 3-(4) | 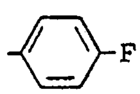 | Powder<br>FABMS (m/z): 513 ($M^+$+Na)<br>IR $\nu_{Max}^{Nujol}$ ($cm^{-1}$): 3300, 1600, 1570, 1500 |

| | | |
|---|---|---|
| 4-(4) | 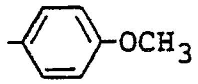 | Powder<br>FABMS (m/z): 525 ($M^+$+Na)<br>IR $\nu_{Max}^{Nujol}$ ($cm^{-1}$): 3450, 1600, 1575, 1508 |
| 5-(4) | 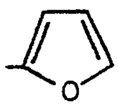 | Powder<br>FABMS (m/z): 485 ($M^+$+Na)<br>IR $\nu_{Max}^{Nujol}$ ($cm^{-1}$): 3336, 1600, 1575, 1506 |
| 6-(4) | 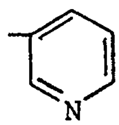 | M.p.: 218-220°C (decomposed)<br>FABMS (m/z): 496 ($M^+$+Na)<br>IR $\nu_{Max}^{Nujol}$ ($cm^{-1}$): 3300, 1598, 1569, 1504 |

Example 7

(1) To a suspension of 62.4 % sodium hydride (0.76 g) in tetrahydrofuran (5 ml) is added dropwise a solution of diethyl 2(cyclohexylamino)vinylphosphonate (3.68 g) in tetrahydrofuran (20 ml) under ice-cooling, and the mixture is stirred for 20 minutes. Thereto is added a solution of 3,3-bis(4-fluorophenyl)-2-(3-pyridyl)acrylaldehyde (2.26 g) in tetrahydrofuran (15 ml), and the mixture is stirred at room temperature for 1 hour, and further stirred at 50°C for 1 hour. The solvent is distilled off from the reaction solution, and thereto is added ice-water, and then extracted with ethyl acetate. The extract is washed with water, dried, and the solvent is distilled off. To the resulting residue are added tetrahydrofuran (50 ml) and 5 % hydrochloric acid (25 ml), and the mixture is refluxed for 45 minutes. The solvent is distilled off, and to the resulting residue is added water, and the mixture is extracted with chloroform. The extract is washed and dried, and the solvent is distilled off. The resulting residue is purified by silica gel column chromatography (solvent; ethyl acetate : n-hexane = 1 : 1) to give (E)-5,5-bis(4-fluorophenyl)-4-(3-pyridyl)-2,4-pentadienal (2.08 g).
Yield: 85 %
M.p.: 129 - 130°C (recrystallized from ethyl acetate/n-hexane)
MS (m/z): 347 ($M^+$), 318

$$IR\ \nu_{Max}^{Nujol}\ (cm^{-1}):\ 1670$$

(2) The product obtained in above (1) is treated in the same manner as in Example 1-(2) to give methyl (E)-9,9-bis(4-fluorophenyl)-5-hydroxy-3-oxo-8-(3-pyridyl)-6,8-nonadienoate as oil.
MS (m/z): 463 ($M^+$), 318

$$IR\ \nu_{Max}^{Nujol}\ (cm^{-1}):\ 3600,\ 1740,\ 1720$$

(3) The product obtained in above (2) is treated in the same manner as in Example 1-(3) to give methyl (3R*,5S*)-(E)-9,9-bis(4-fluorophenyl)-3,5-dihydroxy-8-(3-pyridyl)-6,8-nonadienoate as oil.
MS (m/z): 465 ($M^+$), 318

$$IR\ \nu_{Max}^{Nujol}\ (cm^{-1}):\ 3430,\ 1730$$

(4) The product obtained in above (3) is treated in the same manner as in Example 1-(4) to give sodium (3R*,5S*)-(E)-9,9-bis(4-fluorophenyl)-3,5-dihydroxy-8-(3-pyridyl)-6,8-nonadienoate as powder.
FABMS (m/z): 496 ($M^+$ + Na)

$$IR\ \nu_{Max}^{Nujol}\ (cm^{-1}):\ 3350,\ 1600$$

Example 8

(1) 3,3-Bis(4-fluorophenyl)-2-(2-oxo-1-pyrrolidinyl)acrylaldehyde is treated in the same manner as in Example 7-(1) to give (E)-5,5-bis(4-fluorophenyl)-4-(2-oxo-1-pyrrolidinyl)-2,4-pentadienal.
M.p.: 153.5 - 155 °C
MS (m/z): 353 ($M^+$), 324, 268

$$IR\ \nu_{Max}^{Nujol}\ (cm^{-1}):\ 1700,\ 1670,\ 1600$$

(2) The product obtained in above (1) is treated in the same manner as in Example 1-(2) to give methyl (E)-9,9-bis(4-fluorophenyl)-5-hydroxy-3-oxo-8-(2-oxo-1-pyrrolidinyl)-6,8-nonadienoate as oil.
MS (m/z): 469 ($M^+$), 384

$$IR\ \nu_{Max}^{Nujol}\ (cm^{-1}):\ 3380,\ 1745,\ 1720,\ 1680$$

(3) The product obtained in above (2) is treated in the same manner as in Example 1-(3) to give methyl (3R*,5S*)-(E)-9,9-bis(4-fluorophenyl)-3,5-dihydroxy-8-(2-oxo-1-pyrrolidinyl)-6,8-nonadienoateas oil.
MS (m/z): 471 ($M^+$), 386

$$IR\ \nu_{Max}^{Nujol}\ (cm^{-1}):\ 3350,\ 1735,\ 1670$$

(4) The product obtained in above (3) is treated in the same manner as in Example 1-(4) to give sodium (3R*,5S*)-(E)-9,9-bis(4-fluorophenyl)-3,5-dihydroxy-8-(2-oxo-1-pyrrolidinyl)-6,8-nonadienoate as powder.
FABMS (m/z): 502 ($M^+$ + Na)

$$IR\ \nu_{Max}^{Nujol}\ (cm^{-1}):\ 3360,\ 1680,\ 1600,\ 1575$$

Example 9

(1) To a mixture of sodium (3R*,5S*)-(E)-9,9-bis(4-fluorophenyl)-3,5-dihydroxy-8-phenyl-6,8-nonadienoate (2 g) and ethyl acetate (20 ml) is added 10 % hydrochloric acid under ice-cooling, and the mixture is stirred at room temperature for 10 minutes. The organic layer is separated, and the solvent is distilled off to give (3R*,5S*)-(E)-9,9-bis(4-fluorophenyl)-3,5-dihydroxy-8-phenyl-6,8-nonadienoic acid (1.80 g) as foam.
Yield: 99 %
MS (m/z): 450 ($M^+$), 433

$$IR\ \nu_{Max}^{Nujol}\ (cm^{-1}):\ 3400,\ 1740,\ 1720$$

(2) A mixture of the product (1.5 g) obtained in above (1) and toluene (50 ml) is refluxed by using a reflux condenser (Dean Stark) filled with Molecular Sieves 4A for 3 hours. The solvent is distilled off, and the resulting residue is recrystallized from a mixture of isopropyl ether and n-hexane to give (±)-trans-(E)-6-[4,4-bis(4-fluorophenyl)-3-phenyl-1,3-butadienyl]-3,4,5,6-tetrahydro-4-hydroxy-2H-pyran-2-one (0.98 g) as colorless needles.
Yield: 69 %
M.p.: 135 - 137° C
MS (m/z): 432 ($M^+$), 317

$$IR\ \nu_{Max}^{Nujol}\ (cm^{-1}):\ 3420,\ 1740,\ 1700$$

Example 10

To an aqueous solution of sodium (3R*,5S*)-(E)-9,9-bis(4-fluorophenyl)-3,5-dihydroxy-8-(3-pyridyl)-6,8-nonadienoate (482.5 mg) in water (15 ml) is added 1 N hydrochloric acid (1 ml), and the mixture is extracted with chloroform. The extract is dried and filtered, and the solvent is distilled off. Toluene (50 ml) is added to the residue, and the mixture is refluxed by using a reflux condenser (Dean Stark) filled with Molecular Sieves 4A for 5 hours. The reaction solution is washed, dried and filtered. The solvent is distilled off, and the resulting residue is recrystallized from a mixture of ethyl acetate and n-hexane to give (±)-trans-(E)-6-[4,4-bis(4-fluorophenyl)-3-(3-pyridyl)-1,3-butadienyl]-3,4,5,6-tetrahydro-4-hydroxy-2H-pyran-2-one (341 mg) as light brown prisms.
Yield: 79 %
M.p.: 172 - 172.5° C
MS (m/z): 433 ($M^+$), 318

$$IR\ \nu_{Max}^{Nujol}\ (cm^{-1}):\ 3200,\ 1735$$

Examples 11 - 12

The trans-type compounds of the following Table 6 are obtained by treating the products obtained in Example 2-(3) or in Example 5-(3) in the same manner as in Example 1-(4) and Example 9.

Table 6

| Ex. No. | 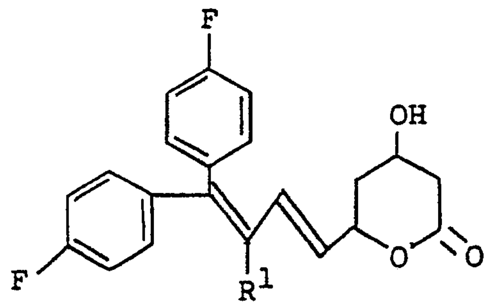 | |
|---|---|---|
| | $R^1$ | Physicochemical Properties |
| 11 | 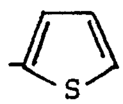 | Light yellow foam<br>MS (m/z): 438 ($M^+$)<br>IR $\nu^{Nujol}_{Max}$ (cm-1): 3410, 1735, 1720, 1600 |
| 12 | 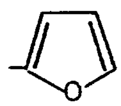 | Yellow foam<br>MS (m/z): 422 ($M^+$)<br>IR $\nu^{Nujol}_{Max}$ ($cm^{-1}$): 3410, 1735, 1720, 1600 |

Example 13

(1) A solution of (±)-trans-(E)-6-[4,4-bis(4-fluorophenyl)-3-(2-thienyl)-1,3-butadienyl]-3,4,5,6-tetrahydro-4-hydroxy-2H-pyran-2-one (21.93 g) and R-(+)-1-phenylethylamine (10.46 g) in toluene (110 ml) is refluxed for 3 hours. After completion of the reaction, to the mixture is added 10 % hydrochloric acid under ice-cooling, and extracted with ethyl acetate. The ethyl acetate layer is washed, dried and filtered. The solvent is distilled off and the resulting residue is subjected to silica gel column chromatography (solvent ; ethyl acetate : n-hexane = 2 : 1). From the first eluent, (3R*,5S*)-(E)-N-((R)-1-phenylethyl)-9,9-bis(4-fluorophenyl)-3,5-dihydroxy-8(2-thienyl)-6,8-nonadienoic acid amide (10.28 g, hereinafter referred to as Compound A) is obtained as colorless caramel.

Compound A

Yield: 37 %
$[\alpha]_D^{20}$ : +38.2° (c = 1.0, chloroform)
MS (m/z): 559 ($M^+$)

$$IR\ \nu^{Nujol}_{Max}\ (cm^{-1}): 3300, 1640, 1600$$

From the subsequent eluent, (5R*,3S*)-(E)-N-((R)-1-phenylethyl)-9,9-bis(4-fluorophenyl)-3,5-dihydroxy-8-(2-thienyl)-6,8-nonadienoic acid amide (11.52 g, hereinafter referred to as Compound B) is obtained as colorless caramel.

Compound B

Yield: 41 %
$[\alpha]_D^{20}$ : +37.6° (c=1.0, chloroform)
MS (m/z): 559 ($M^+$)

$$IR\ \nu_{Max}^{Nujol}\ (cm^{-1}):\ 3300,\ 1640,\ 1600$$

(2) To a solution of Compound A (10.28 g) in ethanol (130 ml) is added an aqueous solution of sodium hydroxide (8 g) in water (30 ml), and the mixture is refluxed under argon atmosphere for 12 hours. After completion of the reaction, the solvent is distilled off, and diluted with ice-water. The mixture is acidified with 10 % hydrochloric acid, and extracted with ethyl acetate. The ethyl acetate layer is washed, dried, and filtered. The solvent is distilled off, and toluene (200 ml) is added to the resulting residue. The mixture is refluxed by using a reflux condenser (Dean Stark) filled with Zeolite A-4 for 7 hours. After cooling, ethyl acetate is added to the mixture, and the mixture is washed, dried, filtered and concentrated to dryness under reduced pressure. The resulting residue is purified by silica gel column chromatography (solvent; ethyl acetate : n-hexane = 1 : 3), and recrystallized from a mixture of ethyl acetate and isopropyl ether to give (+)-trans-(E)-6-[4,4-bis(4-fluorophenyl)-3-(2-thienyl)-1,3-butadienyl]-3,4,5,6-tetrahydro-4-hydroxy-2H-pyran-2-one (3.59 g) as colorless needles.
Yield: 44 %
M.p.: 159 - 161.5° C
$[\alpha]_D^{20}$ : +98.6° (c=1.0, chloroform)
MS (m/z): 438 ($M^+$)

$$IR\ \nu_{Max}^{Nujol}\ (cm^{-1}):\ 3480,\ 1705,\ 1600$$

Compound B (735 mg) is treated in the same manner as above to give (-)-trans-(E)-6-[4,4-bis(4-fluorophenyl)-3-(2-thienyl)-1,3-butadienyl]-3,4,5,6-tetrahydro-4-hydroxy-2H-pyran-2-one (360 mg) as colorless needles.
Yield: 62 %
M.p.: 160.5 - 163° C (recrystallized from ethyl acetate/isopropyl ether)
$[\alpha]_D^{20}$ : -97.7° (c=1.0, chloroform)
MS (m/z): 438 ($M^+$)

$$IR\ \nu_{Max}^{Nujol}\ (cm^{-1}):\ 3480,\ 1705,\ 1600$$

Example 14

To an ice-cooled solution of (+)-trans-(E)-6-[4,4-bis(4-fluorophenyl)-3-(2-thienyl)-1,3-butadienyl]-3,4,5,6-tetrahydro-4-hydroxy-2H-pyran-2-one (2.4 g) in ethanol (30 ml) is added 1 N aqueous sodium hydroxide solution (17 ml), and the mixture is stirred at room temperature for 0.5 hour. Ethanol is distilled off, and the resulting residue is purified by column chromatography filled with nonionic absorption resin [absorbent; Daiaion HP-20 (trade name) manufactured by Mitsubishi Kasei Corporation, solvent; methanol : water = 3 : 2] to give sodium (+)-(3R*,5S*)-(E)-9,9-bis(4-fluorophenyl)-3,5-dihydroxy-8-(2-thienyl)-6,-8-nonadienoate (2.42 g) as light yellow powder.
Yield: 92 %
$[\alpha]_D^{20}$ : +11.1° (c=1.0, methanol)
FABMS (m/z): 501 ($M^+$+Na)

$$IR\ \nu_{Max}^{Nujol}\ (cm^{-1}): 3380, 1600$$

(-)-Trans-(E)-6-[4,4-bis(4-fluorophenyl)-3-(2-thienyl)-1,3-butadienyl]-3,4,5,6-tetrahydro-4-hydroxy-2H-pyran-2-one (360 mg) is treated in the same manner as above to give sodium (-)(3R*,5S*)-(E)-9,9-bis(4-fluorophenyl)-3,5-dihydroxy-8-(2-thienyl)-6,8-nonadienoate (254 mg) as colorless powder.
Yield: 64 %
$[\alpha]_D^{20}$ : -11.0° (c=1.0, methanol)
FABMS (m/2): 501 ($M^+$ + Na)

$$IR\ \nu_{Max}^{Nujol}\ (cm^{-1}): 3380, 1600$$

Example 15

(1) (±)-Trans-(E)-6-[4,4-bis(4-fluorophenyl)-3-(2-furyl)-1,3-butadienyl]-3,4,5,6-tetrahydro-4-hydroxy-2H-pyran-2-one (1.68 g) is treated in the same manner as in Example 13-(1), and further subjected to silica gel column chromatography (solvent; ethyl acetate : n-hexane = 2 : 3). From the first eluent, (3R*,5S*)-(E)-N-((R)-1-phenylethyl)-9,9-bis(4-fluorophenyl)-3,5-dihydroxy-8-(2-furyl)-6,8-nonadienoic acid amide (660 mg, hereinafter referred to as Compound C) is obtained as light yellow foam.

Compound C

Yield: 30 %
MS (m/z): 543 ($M^+$)

$$IR\ \nu_{Max}^{Nujol}\ (cm^{-1}): 3300, 1640$$

From the subsequent eluent, (5R*,3S*)-(E)-N-((R)-1-phenylethyl)-9,9-bis(4-fluorophenyl)-3,5-dihydroxy-8-(2-furyl)-6,8-nonadienoic acid amide (950 mg, hereinafter referred to as Compound D) is obtained as light yellow foam.

Compound D

Yield: 44 %
MS (m/z): 543 ($M^+$)

$$IR\ \nu_{Max}^{Nujol}\ (cm^{-1}): 3300, 1640$$

(2) Compound C (630 mg) is treated in the same manner as Example 13-(2) to give (+)-trans-(E)-6-[4,4-bis(4-fluorophenyl)-3-(2-furyl)-1,3-butadienyl]-3,4,5,6-tetrahydro-4-hydroxy-2H-pyran-2-one (330 mg) as light yellow crystals.
Yield: 67 %
M.p.: 160 - 162° C (recrystallized from ethyl acetate/n-hexane)
$[\alpha]_D^{20}$ : +102.5° (c=1.09, chloroform)
MS (m/z): 422 ($M^+$)

$$IR\ \nu_{Max}^{Nujol}\ (cm^{-1}): 3480, 1710, 1600$$

Compound D (920 mg) is treated in the same manner as above to give (-)-trans-(E)-6-[4,4-bis(4-fluorophenyl)-3-(2-furyl)-1,3-butadienyl]-3,4,5,6-tetrahydro-4-hydroxy-2H-pyran-2-one (440 mg) as light yellow foam.
Yield: 62 %
$[\alpha]_D^{20}$ : -98.5° (c=1.12, chloroform)
MS (m/z): 422 ($M^+$)

$$IR\ \nu_{Max}^{Nujol}\ (cm^{-1}):\ 3420,\ 1730$$

Example 16

(+)-Trans-(E)-6-[4,4-bis(4-fluorophenyl)-3-(2-furyl)-1,3-butadienyl]-3,4,5,6-tetrahydro-4-hydroxy-2H-pyran-2-one (310 mg) is treated in the same manner as in Example 14 to give sodium (-)-(3R*,5S*)-(E)-9,9-bis(4-fluorophenyl)-3,5-dihydroxy-8-(2-furyl)-6,8-nonadienoate (280 mg) as colorless powder.
Yield: 83 %
$[\alpha]_D^{20}$ : -8.0° (c=1.0, water)
FABMS (m/z): 485 ($M^+$+Na), 463 ($M^+$+H)

$$IR\ \nu_{Max}^{Nujol}\ (cm^{-1}):\ 3360,\ 1570$$

(-)-Trans-(E)-6-[4,4-bis(4-fluorophenyl)-3-(2-furyl)-1,3-butadienyl]-3,4,5,6-tetrahydro-4-hydroxy-2H-pyran-2-one (430 mg) is treated in the same manner as above to give sodium (+)-(3R*,5S*)-(E)-9,9-bis(4-fluorophenyl)-3,5-dihydroxy-8-(2-furyl)-6,8-nonadienoate (350 mg) as colorless powder.
Yield: 74 %
$[\alpha]_D^{20}$ : +7.8° (c=1.0, water)
FABMS (m/z): 485 ($M^+$+Na), 463 ($M^+$+H)

$$IR\ \nu_{Max}^{Nujol}\ (cm^{-1}):\ 3360,\ 1570$$

Preparation of the starting compounds

Reference Example 1

(1) A mixture of benzyl chloride (1.9 g), magnesium (0.37 g), ether (13 ml) and a very small amount of iodine is stirred, and thereto is added dropwise a solution of 4,4'-difluorobenzophenone (2.18 g) in ether (10 ml) and tetrahydrofuran (5 ml) under ice-cooling. After the addition, the mixture is stirred at room temperature for 10 minutes, and cooled to 0° C. Thereto is added a saturated aqueous ammonium chloride solution, and extracted with ethyl acetate. The extract is washed and dried, and the solvent is distilled off to give 1,1-bis(4-fluorophenyl)-2-phenylethyl alcohol (3.36 g) as oily product. To this product are added toluene (40 ml) and p-toluenesulfonic acid monohydrate (400 mg), and the mixture is refluxed for 1 hour. After completion of the reaction, water is added to the mixture, and the mixture is extracted with ethyl acetate. The extract is washed and dried, and the solvent is distilled off to give 1,1-bis(4-fluorophenyl)-2-phenylethylene (3.04 g), m.p. 88 - 90° C.
(2) A mixture of the product (3.0 g) obtained in above (1) and acetic acid (20 ml) is kept at a temperature below 40° C, and thereto is added dropwise bromine (1.88 g). After completion of the addition, thereto is added water, and extracted with ethyl acetate, and the solvent is distilled off. The resulting crude crystal is recrystallized from n-hexane to give 1-bromo-2,2-bis(4-fluorophenyl)-1-phenylethylene (2.65 g) as light brown needles, m.p. 103 - 103.5° C.

Reference Examples 2 - 3

The compounds of the following Table 7 are obtained by treating (4-fluorophenyl)methyl chloride or (4-methoxyphenyl)methyl chloride in the same manner as in Reference Example 1.

Table 7

| Ref. Ex. No. | 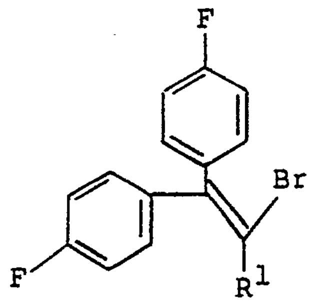 | |
|---|---|---|
| | $R^1$ | Physicochemical Properties |
| 2 | 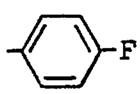 | M.p.: 113-115°C |
| 3 | 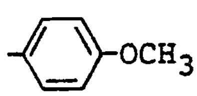 | Oil<br>IR $\nu_{Max}^{Nujol}$ ($cm^{-1}$): 1600, 1500 |

Reference Example 4

(1) To a mixture of 4,4-difluorobenzophenone (10.9 g), ethyl bromoacetate (16.7 g), zinc powder (8.2 g) and a mixture (80 ml) of benzene : toluene [1 : 1 (v/v)] is added a very small amount of iodine, and the mixture is refluxed. After 45 minutes, the reaction solution is poured into a mixture of ice and diluted sulfuric acid. The mixture is filtered, and the filtrate is extracted with ethyl acetate, washed, dried, and filtered, and the solvent is distilled off. To the resulting yellow solid are added ethanol (150 ml), potassium hydroxide (10 g) and water (40 ml), and the mixture is refluxed for 1 hour. After completion of the reaction, the solvent is distilled off, and water is added thereto. The mixture is washed with ether, and poured into a mixture of ice and diluted sulfuric acid. The precipitated crystal is collected by filtration, and dried to give light brown solid (12.8 g). This product is dissolved in ethanol (200 ml), and thereto is added dropwise thionyl chloride (25 ml) under ice-cooling. The mixture is stirred at the same temperature for 1 hour, and further stirred at room temperature for 1 hour, and then refluxed for 1 hour. After completion of the reaction, the solvent is distilled off, and the resulting residue is purified and separated by silica gel column chromatography (solvent; ethyl acetate : n-hexane = 1 : 15) to give ethyl 3,3-bis(4-fluorophenyl)acrylate (12.8 g) as light yellow solid, m.p. 60 -61 ° C.

(2) This product is treated in the same manner as in Reference Example 1-(2) to give ethyl 2-bromo-3,3-bis(4-fluorophenyl)acrylate, m.p. 78 - 79 ° C.

Reference Example 5

(1) A mixture of 1-bromo-2,2-bis(4-fluorophenyl)-1-phenylethylene (2.8 g) and ether (28 ml) is stirred at -50 ° C, and thereto is added a 1.6 M solution of n-butyllithium in n-hexane (7 ml) and stirred at the same temperature for 10 minutes. To the reaction mixture are added successively dimethylformamide (820

mg) and a saturated aqueous ammonium chloride solution, and the mixture is extracted with ethyl acetate. The solvent is distilled off, and the resulting residue is purified by silica gel column chromatography (solvent; n-hexane : ethyl acetate = 15 : 1), and recrystallized from a mixture of ethyl acetate and n-hexane to give 3,3-bis(4-fluorophenyl)-2-phenylacrylaldehyde (2.12 g) as light yellow needles, m.p. 147.5 - 148° C.

(2) A mixture of 60 % sodium hydride (187 mg) and tetrahydrofuran (14 ml) is cooled in an ice-bath, and thereto is added dropwise a solution of diisopropyl cyanomethylphosphate (945 mg) in tetrahydrofuran (3 ml), and the mixture is stirred at room temperature for 20 minutes. The reaction mixture is cooled again, and thereto is added dropwise a solution of 3,3-bis(4-fluorophenyl)-2-phenyl-acrylaldehyde (1.23 g) in tetrahydrofuran (3 ml). The mixture is stirred at room temperature for 5 minutes, and thereto is added water, and extracted with ethyl acetate. The solvent is distilled off, and the resulting residue is purified by silica gel column chromatography (solvent; n-hexane : ethyl acetate = 20 : 1) to give (E)-5,5-bis(4-fluorophenyl)-4-phenyl-2,4-pentadienenitrile (1.2 g) as colorless needles, m.p. 130 - 133° C.

<u>Reference Example 6</u>

(1) A mixture of ethyl 2-bromo-3,3-bis(4-fluorophenyl)acrylate (4.98 g), 2-tri(n-butyl)stannylthiophene (6.07 g), bis(triphenylphosphine)palladium (II) chloride (375 mg) and dioxane (20 ml) is refluxed for 18 hours. After completion of the reaction, thereto is added an aqueous potassium fluoride solution, and the mixture is extracted with ethyl acetate. The extract is filtered, washed and dried. The solvent is distilled off from the filtrate. The resulting residue is purified by silica gel column chromatography (solvent; ethyl acetate : n-hexane = 1 : 9) to give ethyl 3,3-bis(4-fluorophenyl)-2-(2-thienyl)acrylate (4.14 g) as yellow solid, m.p. 77.5 - 80° C.

(2) A mixture of the product (1.0 9) obtained in above (1), methylene chloride (10 ml) and 1.5 M solution of diisobutylaluminum hydride in toluene (5.4 ml) is stirred at 0° C for 10 minutes. After completion of the reaction, thereto are added successively 10 % hydrochloric acid and water, and the mixture is extracted with ethyl acetate. The solvent is distilled off to give 3,3-bis(4-fluorophenyl)-2-(2-thienyl)allyl alcohol (770 mg) as colorless crystal, m.p. 108 - 110° C.

(3) A mixture of the product (2.31 g) obtained in above (2), pyridinium chlorochromate (2.3 g) and methylene chloride (23 ml) is stirred at room temperature for 40 minutes. The mixture is extracted with ether and the organic layer is separated by decantation, and the solvent is distilled off. The resulting residue is purified and separated by silica gel column chromatography (solvent; n-hexane : ethyl acetate = 5 : 1) to give 3,3-bis(4-fluorophenyl)-2-(2-thienyl)acrylaldehyde (1.75 g) as yellow leaflets, m.p. 143 - 145 ° C.

(4) The product obtained in above (3) is treated in the same manner as in Reference Example 5-(2) to give (E)-5,5-bis(4-fluorophenyl)-4-(2-thienyl)-2,4-pentadiene-nitrile, m.p. 167 - 169° C.

<u>Reference Examples 7 - 10</u>

The compounds of the following Table 8 are obtained in the same manner as in Reference Example 5 or 6.

Table 8

| Ref. Ex. No. | 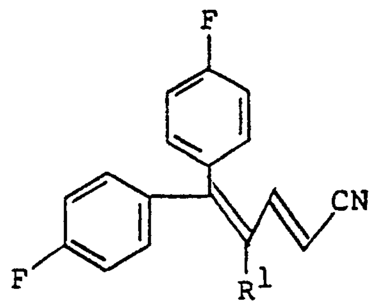 | |
|---|---|---|
| | $R^1$ | Physicochemical Properties |
| 7 | 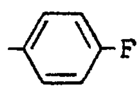 | M.p. 158-161°C |
| 8 | 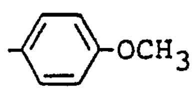 | M.p. 158-159°C |
| 9 | 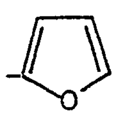 | M.p. 104-108°C |
| 10 | 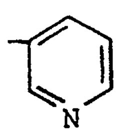 | M.p. 141.5-144°C |

Reference Example 11

Ethyl 2-bromo-3,3-bis(4-fluorophenyl)acrylate and 3-tri(n-butyl)stannylpyridine are treated in the same manner as in Reference Example 6-(1) - (3) to give 3,3-bis(4-fluorophenyl)-2-(3-pyridyl)acrylaldehyde, m.p. 118 - 120° C.

Reference Example 12

(1) To a mixture of ethyl 2-bromo-3,3-bis(4-fluorophenyl)acrylate (11 g) and tetrahydrofuran (60 ml) is added dropwise a 1.5 M solution of diisopropylaluminium hydride in toluene (40 ml) at 0 - 5° C under argon atmosphere, and the mixture is stirred at room temperature for 30 minutes. The reaction mixture is poured into 10 % hydrochloric acid and ice, and extracted with ethyl acetate. The solvent is distilled off to give 2-bromo-3,3-bis(4-fluorophenyl)prop-2-en-l-ol(9.77 g) as oil.

$$\text{IR } \nu_{\text{Max}}^{\text{Liquid}} (\text{cm}^{-1}): 3377, 1601, 1506$$

(2) A mixture of the product (9.77 g) obtained in above (1), 2-pyrrolidone (40 ml) and copper powder (20 g) is heated with stirring at 150° C under argon atmosphere for 55 minutes. After cooling, thereto are added water and ethyl acetate. The insoluble materials are removed by filtration, and the organic layer is separated. The solvent is distilled off, and the resulting residue is purified by silica gel column chromatography (solvent; chloroform : methanol = 40 : 1), and recrystallized from a mixture of ethyl acetate and n-hexane to give 3,3-bis(4-fluorophenyl)-2-(2-oxo-1-pyrrolidinyl)prop-2-en-1-ol (7.16 g) as colorless needles, m.p. 129 - 131° C.

(3) A mixture of the product (6.5 g) obtained in above (2), manganese dioxide (21.2 g) and methylene chloride (65 ml) is refluxed for 2 hours. Insoluble materials are removed by filtration, and the solvent is distilled off. The resulting residue is recrystallized from n-hexane to give 3,3-bis(4-fluorophenyl)-2-(2-oxo-1-pyrrolidinyl)acrylaldehyde (6.19 g) as yellow needles, m.p. 107 - 109° C.

## Claims

1. A phenylethylene derivative of the formula:

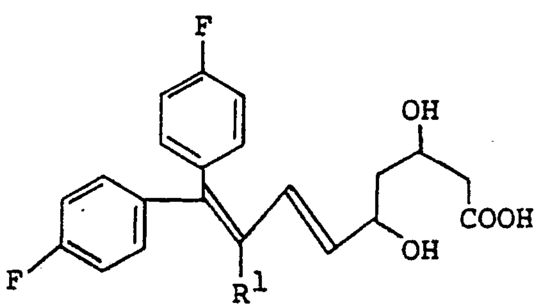

wherein $R^1$ is phenyl group, a fluorophenyl group, a methoxyphenyl group, thienyl group, furyl group, oxopyrrolidinyl group or pyridyl group, or a pharmaceutically acceptable ester, amide, lactone or salt thereof.

2. The compound according to claim 1, which has the formula:

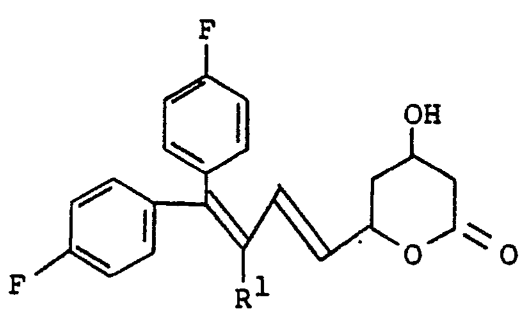

wherein $R^1$ is phenyl group, a fluorophenyl group, a methoxyphenyl group, thienyl group, furyl group,

oxopyrrolidinyl group or pyridyl group.

3. Trans-(E)-6-[4,4-bis(4-fluorophenyl)-3-(2-furyl)-1,3-butadienyl]-3,4,5,6-tetrahydro-4-hydroxy-2H-pyran-2-one.

4. Trans-(E)-6-[4,4-bis(4-fluorophenyl)-3-(2-thienyl)-1,3-butadienyl]-3,4,5,6-tetrahydro-4-hydroxy-2H-pyran-2-one.

5. The compound according to any one of claim 1, 2, 3 or 4, which is an optically active compound.

6. A process for preparing a phenylethylene derivative of the formula:

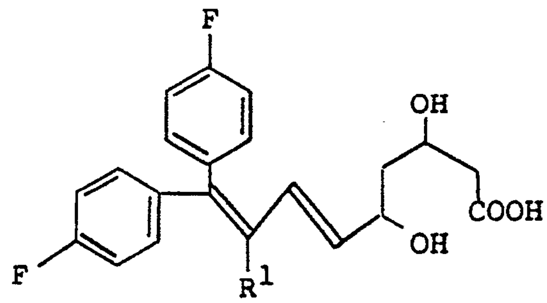

wherein $R^1$ is phenyl group, a fluorophenyl group, a methoxyphenyl group, thienyl group, furyl group, oxopyrrolidinyl group or pyridyl group, or a pharmaceutically acceptable ester, amide, lactone or salt thereof,
which comprises reducing a compound of the formula:

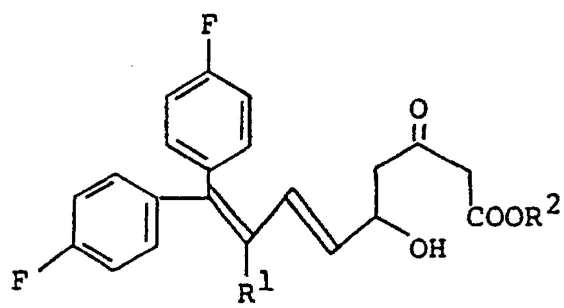

wherein $R^1$ is the same as defined above, and a group of the formula: $-COOR^2$ is a carboxyl group which may optionally have a protecting group, or a salt thereof,
when a group of the formula: $-COOR^2$ is a carboxyl group having a protecting group, if desired, removing the protecting group thereof, and
further, if necessary, converting the product into a pharmaceutically acceptable ester, amide, lactone or salt thereof.

7. A process for preparing a phenylethylene derivative of the formula:

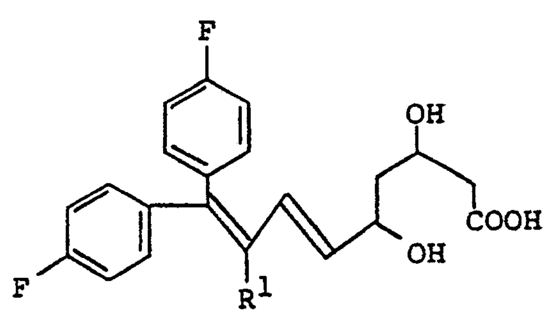

wherein $R^1$ is phenyl group, a fluorophenyl group, a methoxyphenyl group, thienyl group, furyl group, oxopyrrolidinyl group or pyridyl group, or a pharmaceutically acceptable ester, amide, lactone or salt thereof,
which comprises reacting an aldehyde compound of the formula:

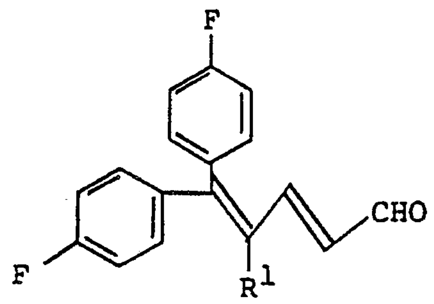

wherein $R^1$ is the same as defined above, or a salt thereof with an acetoacetic acid compound of the formula:

$CH_3COCH_2COOR^2$

wherein a group of the formula: $-COOR^2$ is a carboxyl group which may optionally have a protecting group, or a salt thereof to give a compound of the formula:

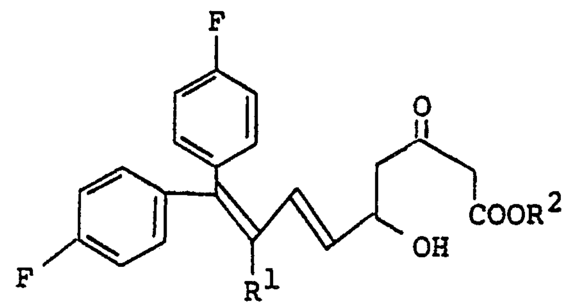

wherein $R^1$ and a group of the formula: $-COOR^2$ are the same as defined above, or a salt thereof, and reducing the product obtained in above or a salt thereof,
when a group of the formula: $-COOR^2$ is a carboxyl group having a protecting, if desired, removing the protecting group thereof, and
if necessary, followed by converting the product into a pharmaceutically acceptable ester, amide, lactone or salt thereof.

**8.** An aldehyde compound of the formula:

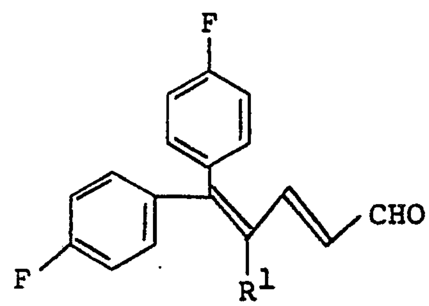

wherein $R^1$ is phenyl group, a fluorophenyl group, a methoxyphenyl group, thienyl group, furyl group, oxopyrrolidinyl group or pyridyl group, or a salt thereof.

9. A pharmaceutical composition which comprises an effective amount of the phenylethylene derivative as set forth in claim 1 as an active ingredient in admixture with a pharmaceutically acceptable carrier or diluent.